# EUROPEAN PATENT APPLICATION

(11) **EP 2 679 140 A1**
(43) Date of publication of application: **01.01.2014**
(21) Application number: 12862379.0
(22) Date of filing: 10.12.2012
(51) Int. Cl.: A61B 1/00, A61B 17/00, A61B 19/00

(54) **MEDICAL ENDOSCOPE SYSTEM**

(30) Priority: 26.12.2011 JP 2011284117
(71) Applicant: Olympus Medical Systems Corp., Tokyo 151-0072 (JP)
(72) Inventor: NEMOTO, Iori, Tokyo 151-0072 (JP); SEKIGUCHI, Kiyoshi, Tokyo 151-0072 (JP)
(74) Representative: Gunzelmann, Rainer
(86) International application number: PCT/JP2012/081982
(87) International publication number: WO 2013/099580

(57) **Abstract**

Medical endoscope system includes image pickup device and control target devices and controller configured to control the control target devices. The controller includes manipulator setting unit configured to set one candidate as manipulator who is permitted to perform manipulation from among candidates registered beforehand and to set the others as manipulation prohibited persons, object detection unit configured to detect object meeting prescribed condition in image from the pickup device, manipulator recognition unit configured to determine object that corresponds to a manipulator set by the manipulator setting unit from among the objects detected by the object detection unit and to recognize the determined object as the manipulator, action detection unit configured to detect prescribed action of the determined object, control signal generation unit configured to generate control signal that controls at least one control target device among the plurality of control target devices in accordance with detection result by the action detection unit, and device control unit configured to control the at least one control target device in accordance with the control signal.

## Description

### Technical Field

The present invention is related to a medical endoscope system that controls, through a controller, a plurality of devices such as an electrocautery scalpel device, a pneumoperitoneum apparatus, an endoscopic camera device, etc.

### Background Art

In the field of medicine, medical endoscope systems having a controller that controls a plurality of devices have conventionally been known. In this type of a medical endoscope system, arbitrary devices in the system can be manipulated through a controller by manipulating a manipulation panel connected to the controller.

Usually, a manipulation panel connected to a controller is disposed in a non-sterilization area within a surgery room, and accordingly it is not appropriate in view of sanitation for a surgeon (a physician for example) to manipulate the manipulation panel directly. Accordingly, it has been common in a conventional medical endoscope system for an assistant (a nurse for example), who assists a surgeon, to manipulate a manipulation panel in accordance with instructions from the surgeon so as to manipulate devices.

However, a problem has been pointed out wherein it is difficult to manipulate devices at timings that a surgeon desires without delays when the manipulation of devices is performed through an assistant.

As techniques related to the above problem, there are gesture techniques. Gesture techniques are disclosed by, for example, Patent Document 1 and Patent Document 2, and have also been applied to medical endoscope systems in recent years.

Applying a gesture technique to a medical endoscope system enables surgeons to manipulate devices directly, not through assistants, thereby making it possible to manipulate devices at desired timings.

Patent Document 1: Japanese Laid-open Patent Publication No. 2004-289850
Patent Document 2: International Publication Pamphlet No. 03/025859,

### Disclosure of the Invention

### Problem to be solved by the Invention

Although applying a gesture technique to a medical endoscope system may be very profitable as described above, problems specific to a gesture technique have been pointed out. Specifically, a camera installed in a medical endoscope system is used for recognizing gestures, and when there are a plurality of surgeons in the viewing field of the camera, the controller sometimes responds to actions of a surgeon that are not intended to manipulate devices.

Usually, during a surgery, only a particular surgeon who leads the surgery wants to perform manipulation of devices such as changing of setting values of the devices. When it is taken into consideration that other surgeons do not perform manipulation of devices against the will of that particular surgeon, a medical endoscope system needs to prohibit manipulation of devices by persons other than that particular surgeon so as to reliably block the control of devices based on the above misrecognition.

However, according to the technique of Patent Document 1 or 2, it is difficult to sufficiently block the control of devices based on misrecognition.

Patent Document 1 describes a technique about a controlling method that enables smooth manipulation or the like by determining relationship between devices and people and by using movement/posture/state of people. More specifically, a technique has been disclosed in which one person is selected from among a plurality of people on the basis of a prescribed priority order of position/posture/person, and postures and motions of that person control devices.

Patent Document 2 discloses a technique in which gestures are recognized while individuals are identified and thereby interfaces corresponding to individuals are provided regardless of positions in a room. More specifically, it is a technique in which individuals are identified from the target's head and height and manipulation commands are transmitted to a manipulation target that is registered beforehand by a hand sign in a room.

Neither of the techniques disclosed by Patent Documents 1 and 2 is a technique that allows only one prescribed particular person to manipulate devices while clearly prohibiting other people from doing so. Accordingly, it is difficult to block control of devices based on misrecognition at a sufficiently high level required for medical endoscope systems.

In view of the above situation, it is an object of the present invention to provide a medical endoscope system that allows only one particular surgeon to manipulate devices with gestures, prohibiting other people from manipulating devices with gestures.

### Means for solving the problems

A medical endoscope system according to a first aspect of the present invention is a medical endoscope system in which the system includes: an image pickup device, a plurality of control target devices, and a controller that controls the plurality of control target devices, and the controller includes: a manipulator setting unit configured to set one manipulator candidate as a manipulator who is permitted to perform manipulation from among a plurality of manipulator candidates registered beforehand, and to set other manipulator candidates other than the one manipulator candidate as manipulation prohibited persons who are prohibited from performing manipulation, an object detection unit configured to detect an object that meets a prescribed condition from an image output from the image pickup device, a manipulator recognition unit configured to determine an object that corresponds to a manipulator set by the manipulator setting unit from among objects detected by the object detection unit, and to recognize the determined object as the manipulator, an action detection unit configured to detect a prescribed action of the determined object, a control signal generation unit configured to generate a control signal that controls at least one control target device among the plurality of control target devices in accordance with a result of detection by the action detection unit, and a device control unit configured to control the at least one control target device in accordance with the control signal.

A second aspect of the present invention is the medical endoscope system according to the first aspect, in which the controller includes a manipulator addition unit configured to change, to a manipulator, a manipulation prohibited person set by the manipulator setting unit.

A third aspect of the present invention is the medical endoscope system according to the second aspect, further including a display unit configured to display information about the medical endoscope system; and an input unit configured to receive an input from outside of the medical endoscope system, in which the manipulator addition unit is configured to change, to a manipulator, a manipulation prohibited person set by the manipulator setting unit in accordance with an input from the input unit in a situation where a window for manipulating each of the plurality of control target devices is displayed on the display unit.

A fourth aspect of the present invention is the medical endoscope system according to the first aspect, further including a storage unit configured to store model information of a portion of the object as the prescribed condition, in which the object detection unit detects the object that meets the prescribed condition from an image output from the image pickup device by referring to the model information stored in the storage unit.

A fifth aspect of the present invention is the medical endoscope system according to the fourth aspect, in which the storage unit further stores identification information for identifying the object; and the manipulator recognition unit determines, by referring to the identification information stored in the storage unit and from among objects detected by the object detection unit, an object that corresponds to a manipulator set by the manipulator setting unit, and recognizes the determined object as the manipulator.

A sixth aspect of the present invention is the medical endoscope system according to the fifth aspect, in which the storage unit further stores gesture information that is information related to the prescribed action; and the action detection unit detects the prescribed action of the determined object by referring to the gesture information stored in the storage unit.

### Effect of the invention

According to the present invention, it is possible to provide a medical endoscope system that permits only one particular surgeon to manipulate a device with gestures while preventing other surgeons from manipulating devices with gestures.

### BRIEF DESCRIPTION OF THE DRAWINGS

FIG. 1 shows an overall configuration of a medical endoscope system according to example 1 of the present invention;
FIG. 2 shows a configuration of the system controller included in the medical endoscope system of the example 1 of the present invention;
FIG. 3 explains data stored in a storage unit included in the medical endoscope system of example 1 of the present invention;
FIG. 4 shows part of processes performed by an action detection unit included in the medical endoscope system of example 1 of the present invention;
FIG. 5A shows window transition in a manipulator setting process of the medical endoscope system of example 1;
FIG. 5B shows a variation example of window transition in the manipulator setting process of the medical endoscope system of example 1;
FIG. 6 is a flowchart explaining a manipulator setting process of the medical endoscope system of example 1;
FIG. 7 shows an example of a situation in which manipulators have been set after the manipulator setting process exemplified in FIG. 6 was performed;
FIG. 8 is a flowchart explaining a device control process of the medical endoscope system of example 1;
FIG. 9A shows a scene in a surgery room when the device control process exemplified in FIG. 8 is being performed;
FIG. 9B shows an image pickup scope of a gesture camera included in the medical endoscope system of example 1;
FIG. 10 shows a configuration of a system controller included in the medical endoscope system of example 2 of the present invention;
FIG. 11 shows window transition in a manipulator addition process for the medical endoscope system of example 2;
FIG. 12 is a flowchart showing the manipulator addition process for the medical endoscope system of example 2; and
FIG. 13 shows an example of a setting state of manipulators before and after the manipulator setting process exemplified in FIG. 12 was performed.

### EMBODIMENTS FOR IMPLEMENTING THE INVENTION

### Example 1

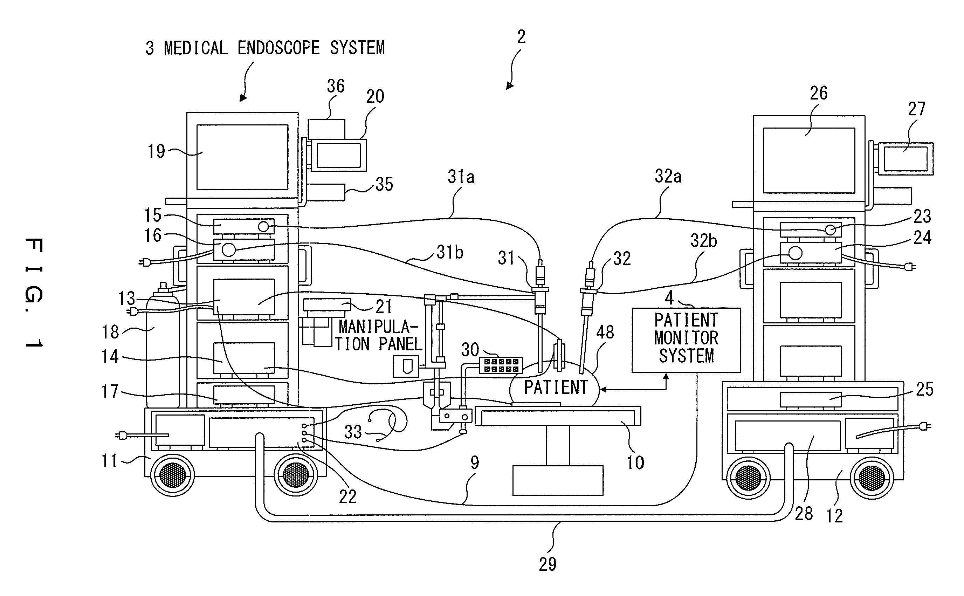

FIG. 1 shows an overall configuration of a medical endoscope system according to the present example. First, a configuration of a medical endoscope system 3 according to the present example will be explained schematically by referring to FIG. 1.

The medical endoscope system 3 exemplified in FIG. 1 is installed in a surgery room 2 together with a patient bed 10, on which a patient 48 is to be laid.

The medical endoscope system 3 includes an electrocautery scalpel device 13, a pneumoperitoneum apparatus 14, an endoscopic camera device 15, a light source device 16, a video tape recorder 17, a gas tank 18, a display device 19, a concentrated display panel 20, a manipulation panel 21, a system controller 22, an RFID (Radio Frequency Identification) terminal 35, and a gesture camera 36, which are mounted on a cart 11.

The medical endoscope system 3 also includes an endoscopic camera device 23, a light source device 24, an image processing device 25, a display device 26, a concentrated display panel 27, and a relay unit 28, which are mounted on a cart 12.

Further, the medical endoscope system 3 includes a patient monitor system 4 connected to a system controller 22 through a cable 9, a remote controller 30, an endoscope 31 that is connected to the endoscopic camera device 15 through a camera cable 31a and that is also connected to the light source device 16 through a light guide cable 31b, an endoscope 32 that is connected to the endoscopic camera device 23 through a camera cable 32a and that is also connected to the light source device 24 through a light guide cable 32b, and a headset-type microphone 33 connected to the system controller 22.

The gas tank 18 has been charged with carbon dioxide. The display device 19 is, for example, a TV monitor, and is configured to display endoscopic images or the like obtained from the endoscope 31. The display device 26 is configured to display endoscopic images or the like obtained from the endoscope 32. The concentrated display panel 20 and the concentrated display panel 27 are configured to selectively display all pieces of data that are to be displayed during a surgery. The manipulation panel 21 is a concentrated manipulation device manipulated by a nurse in a non-sterilization area, and includes a display unit such as a liquid crystal display for displaying information about the medical endoscope system 3 and a touch sensor that is provided to this display unit in an integrated manner so as to receive inputs from outside of the medical endoscope system 3.

The RFID terminal 35 is configured to wirelessly exchange ID information of devices with ID tags embedded in devices such as the endoscope 31, the electrocautery scalpel device 13, and the like. The gesture camera 36 is configured to output images obtained by photographing scenes in the surgery room 2.

The system controller 22 is connected, through a signal line (not shown), to an arbitrary device mounted on the cart 11. The relay unit 28 is connected, through a signal line (not shown) to an arbitrary device mounted on the cart 12, while the system controller 22 is connected to the relay unit 28 through a relay cable 29.

The medical endoscope system 3 configured as described above is capable of controlling, in the methods described below, arbitrary devices connected to the system controller 22.

In the first method, the system controller 22 recognizes gestures of a particular surgeon (operating surgeon for example) on the basis of an image of the surgery room 2 output from the gesture camera 36, and controls arbitrary devices.

In the second method, the system controller 22 makes the display unit of the manipulation panel 21 display a GUI window for displaying manipulation buttons for manipulating arbitrary devices connected to the system controller 22 and setting values or the like of devices, and thereby detects manipulations on the manipulation panel 21 by nurses or the like in order to control arbitrary devices.

In the third method, the system controller 22 detects manipulations on the remote controller 30 by a surgeon (operating surgeon for example), and controls arbitrary devices.

In the fourth method, the system controller 22 recognizes voices of a surgeon (operating surgeon for example) input through the microphone 33, and controls arbitrary devices.

Hereinafter, the method in which arbitrary devices are controlled on the basis of gestures (first method) will be explained in more detail.

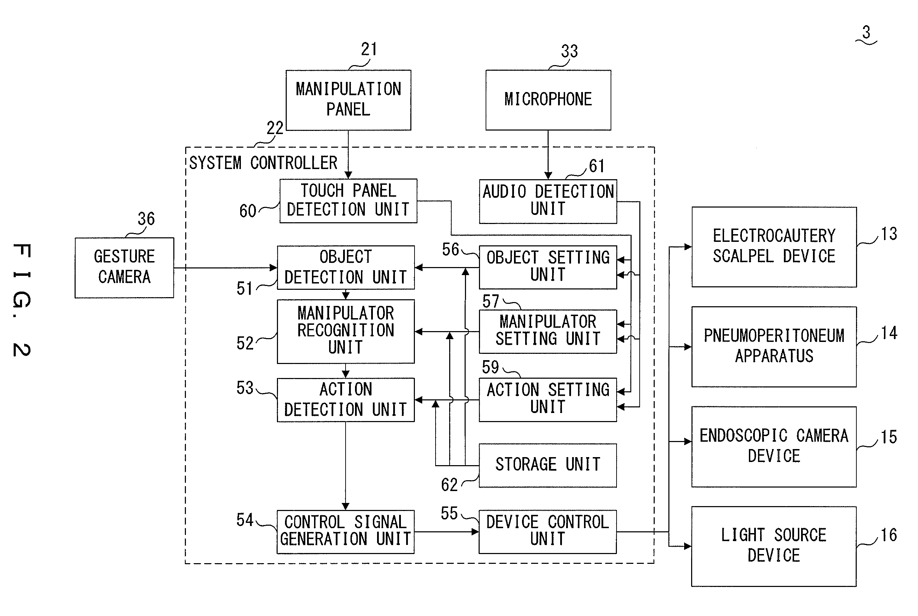

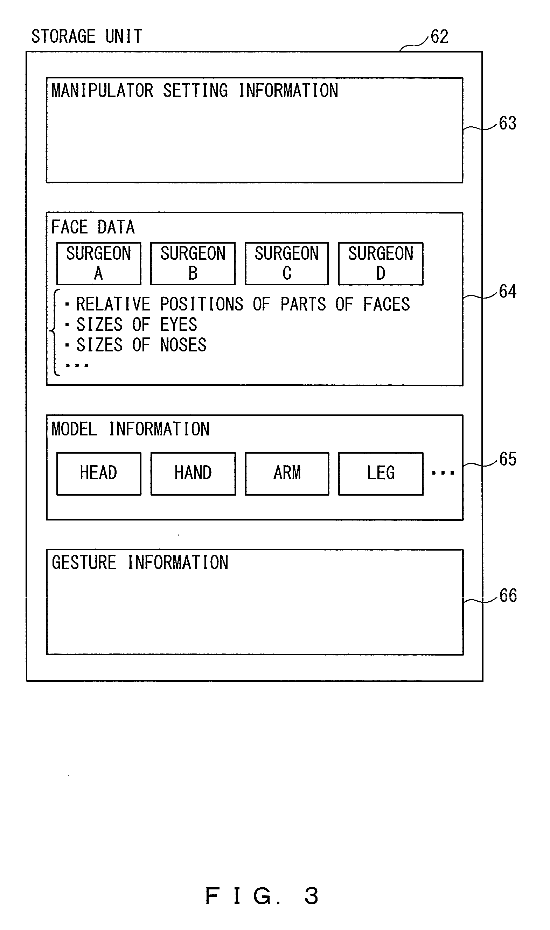

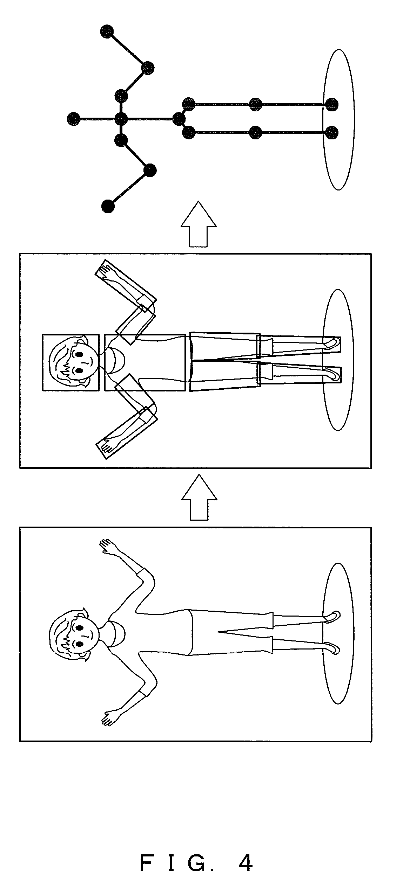

First, a configuration of the system controller 22 will be explained by referring to FIGs. 2 through 4. FIG. 2 shows a configuration of the system controller included in the medical endoscope system according to the present example. FIG. 3 explains data stored in a storage unit included in the medical endoscope system according to the present example. FIG. 4 shows part of processes performed by an action detection unit included in the medical endoscope system according to the present example.

As shown in FIG. 2, the medical endoscope system 3 includes the gesture camera 36, which serves as an image pickup device, a plurality of control target devices (the electrocautery scalpel device 13, the pneumoperitoneum apparatus 14 the endoscopic camera device 15, and the light source device 16), the system controller 22 that controls the plurality of control target devices, the manipulation panel 21, and the microphone 33. Also, although FIG. 2 shows the electrocautery scalpel device 13, the pneumoperitoneum apparatus 14, the endoscopic camera device 15, and the light source device 16 as examples of control target devices, control target devices are not limited to them. It is possible to control, as control target devices, arbitrary devices that are connected to the system controller 22.

The system controller 22 includes an object detection unit 51, a manipulator recognition unit 52, an action detection unit 53, a control signal generation unit 54, a device control unit 55, an object setting unit 56, a manipulator setting unit 57, an action setting unit 59, a touch panel detection unit 60, an audio detection unit 61, and a storage unit 62.

The object detection unit 51 is configured to detect an object that meets a prescribed condition in an image output from the gesture camera 36. An object is, for example, a human in the surgery room 2, and a prescribed condition is a portion of an object, an example of which is a human face. In other words, the object detection unit 51 detects a human face included in an image output from the gesture camera 36 so as to detect a surgeon in the surgery room 2. This prescribed condition is stored as model information 65 of a portion of a human body (a portion of an object) in the storage unit 62 as shown in FIG. 3, and is set by the object setting unit 56 to refer to the model information 65 of a particular portion (head in this example) stored in the storage unit 62 in accordance with inputs from the manipulation panel 21 and the microphone 33.

The manipulator recognition unit 52 is configured to determine, from among surgeons detected by the object detection unit 51, a surgeon corresponding to a manipulator having manipulation permission who is set beforehand by the manipulator setting unit 57 and to recognize the determined surgeon as a manipulator. Specifically, the manipulator recognition unit 52 compares images of faces of a plurality of surgeons detected by the object detection unit 51 to exist in the surgery room 2 with the image of the face of the manipulator set by the manipulator setting unit 57, and thereby determines a surgeon who has been set as a manipulator beforehand, and recognizes the surgeon as a manipulator. Note that "manipulator" used herein means a manipulator with manipulation permission unless otherwise indicated. As shown in FIG. 3, manipulators, who have manipulation permission, are stored as manipulator setting information 63 in the storage unit 62. Also, the image of the face of a manipulator is stored as face data 64 of surgeons in the storage unit 62. The face data 64 serves as identification information for identifying surgeons. The manipulator recognition unit 52 refers to the manipulator setting information 63 and the face data 64 of a manipulator, having manipulation permission, who was set by the manipulator setting unit 57.

The action detection unit 53 is configured to detect a prescribed action of a determined surgeon, i.e., a manipulator. A prescribed action is, for example, a hand movement, and more specifically a movement of swinging a hand from right to left or vice versa, a movement of clenching a fist, a movement of opening a fist, a movement of waving a hand, and the like. In other words, the action detection unit 53 detects gestures of a manipulator. More specifically, first, as shown in FIG. 4, the action detection unit 53 detects respective portions (for example, head, hands, arms, legs, and the like) of the manipulator by referring to the model information 65 stored in the storage unit 62, and forms a frame model of a human body by estimating the posture of the manipulator from the detected respective portions. Then, it tracks the motion of the frame model formed from images output on an as-needed basis from the gesture camera 36, and detects an action of the manipulator. As a last step, it determines whether or not the detected action is a prescribed action so as to detect a gesture action. Also, information related to this prescribed action is stored as gesture information 66 in the storage unit 62 as shown in FIG. 3, and is set by the action setting unit 59 to refer to all or part of pieces of the gesture information 66 stored in the storage unit 62 on the basis of inputs from the manipulation panel 21 and the microphone 33.

The control signal generation unit 54 is configured to generate a control signal that controls at least one manipulation target device among a plurality of manipulation target devices in accordance with detection signals from the action detection unit 53. The control signal generation unit 54 generates a signal that controls a device determined by a gesture detected by the action detection unit 53 so that the device behaves in accordance with the gesture.

The device control unit 55 is configured to control at least one manipulation target device in accordance with a control signal from the control signal generation unit 54. In other words, the device control unit 55 controls a control target device such as electrocautery scalpel device 13 in accordance with a gesture detected by the action detection unit 53.

The manipulator setting unit 57 is configured to set one of a plurality of surgeons (i.e., manipulator candidates) who have been registered beforehand, as a manipulator, who is given manipulation permission, and is configured to set other surgeons as persons who are prohibited from performing manipulation.

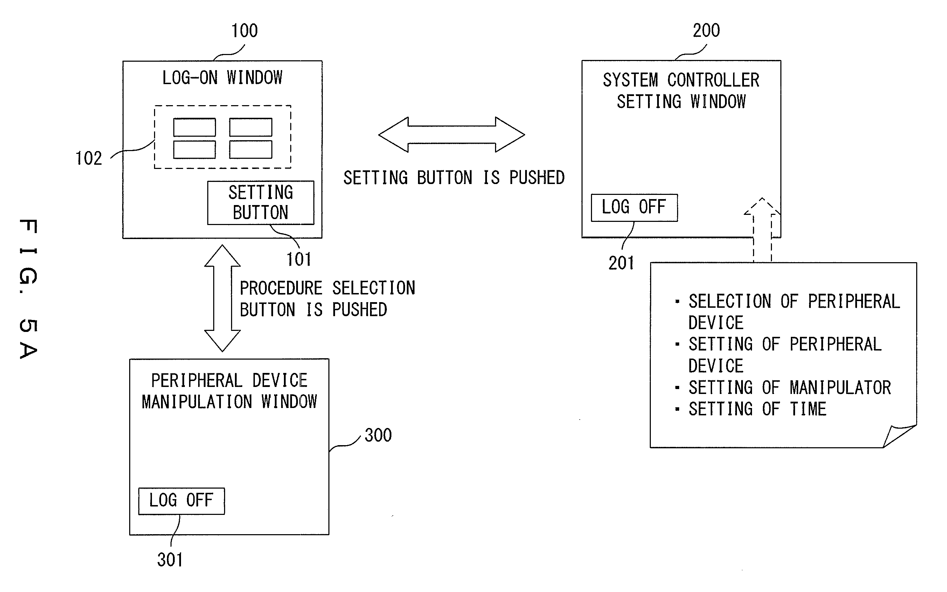

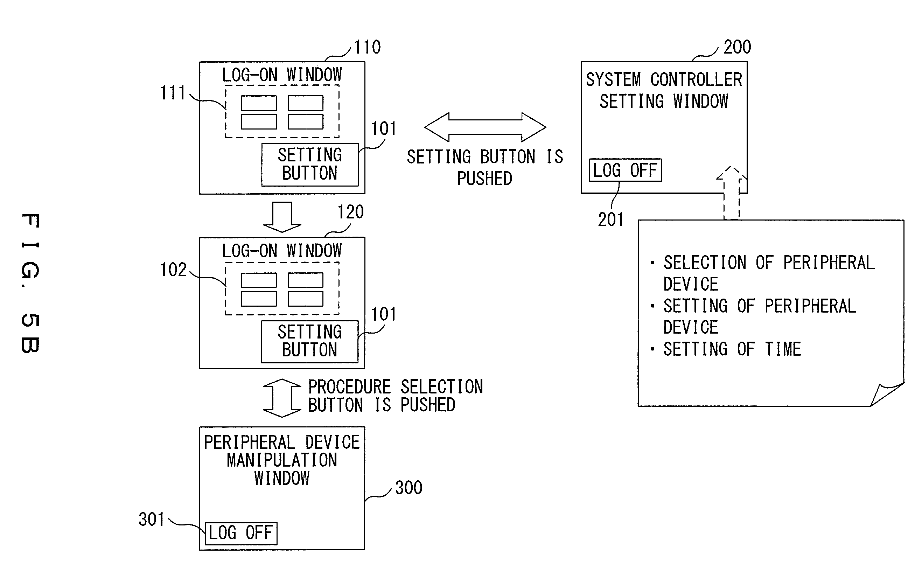

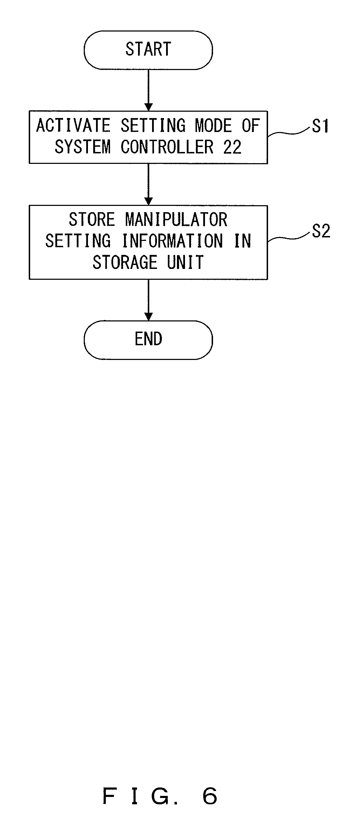

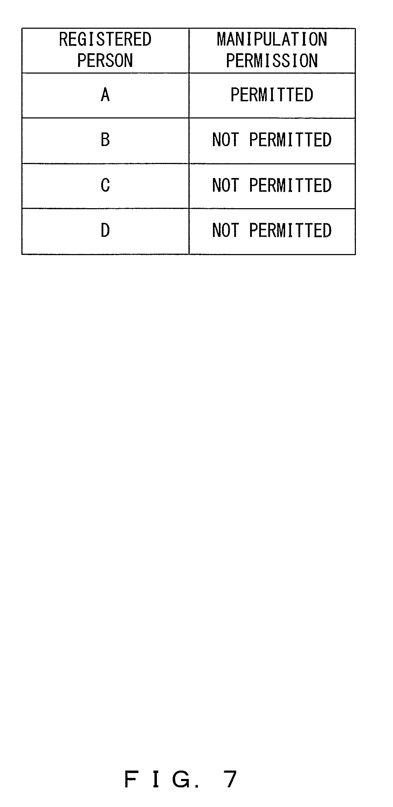

Next, specific explanations will be given for the flow of a manipulator setting process by referring to FIGs. 5A through 7. FIG. 5A shows window transition in a manipulator setting process of the medical endoscope system according to the present example. FIG. 5B shows a variation example of window transition in the manipulator setting process of the medical endoscope system according to the present example. FIG. 6 is a flowchart explaining the manipulator setting process of the medical endoscope system according to the present example. FIG. 7 shows an example of a situation in which manipulators have been set after the manipulator setting process exemplified in FIG. 6.

The manipulator setting process starts when a setting button 101 disposed on a log-on window 100 is pushed while the log-on window 100 exemplified in FIG. 5A is displayed on the manipulation panel 21. The log-on window 100 is provided with a procedure selection button group 102 in addition to the setting button 101. When a button of a procedure is selected in the procedure selection button group 102, the window transitions to a peripheral device manipulation window 300 for manipulating a peripheral device related to the selected procedure.

When the setting button 101 is pushed on the log-on window 100, the window displayed on the manipulation panel 21 transitions to a system controller setting window 200 exemplified in FIG. 5A, and the setting mode of the system controller 22 is activated (S1 in FIG. 6).

On the system controller setting window 200, it is possible to perform various settings related to the medical endoscope system 3, including the setting of a manipulator who is given permission to manipulate devices with gestures. When an input is made to change the setting of a manipulator on the system controller setting window 200, the manipulator setting unit 57 changes the setting of a manipulator in accordance with the input, and manipulator setting information 63 after the change is stored in the storage unit 62 (S2 in FIG. 6).

Also, the setting of a manipulator is performed by selecting one manipulator from among surgeons (registered persons) who are registered in the system controller 22 and whose face data is stored in the storage unit 62. Thereby, a selected surgeon is set as a manipulator, and surgeons other than the selected surgeon are automatically set as manipulation prohibited persons, who are persons prohibited from performing manipulation. FIG. 7 exemplifies a setting state of manipulators who have been registered in the storage unit 62 when surgeon (registered person) A has been selected as a registered person.

Also, the window transition in the manipulator setting process shown in FIG. 5A is an example, and the scope of the present invention is not limited to the example shown in FIG. 5A. For example, it is possible, as shown in FIG. 5B, to employ a configuration in which a surgeon log-in window 110 having manipulator button group 110 and the setting button 101 is first displayed as a first log-in window and when a button for selecting a particular surgeon in the manipulator button group 110 is pushed, the face image of the selected surgeon is displayed, whether or not the selected surgeon is set as a manipulator is confirmed, and the surgeon log-in window 110 having the procedure selection button group 102 and the setting button 101 is displayed as a second log-on window.

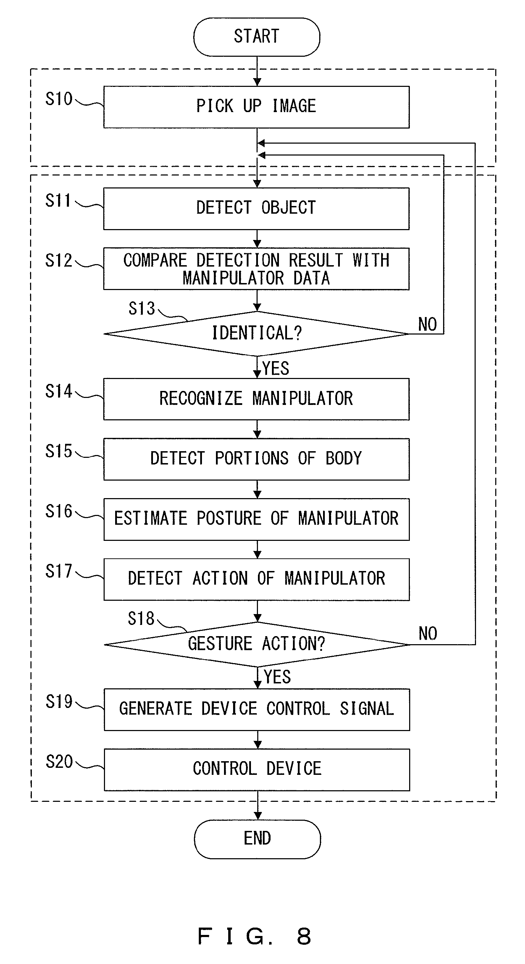

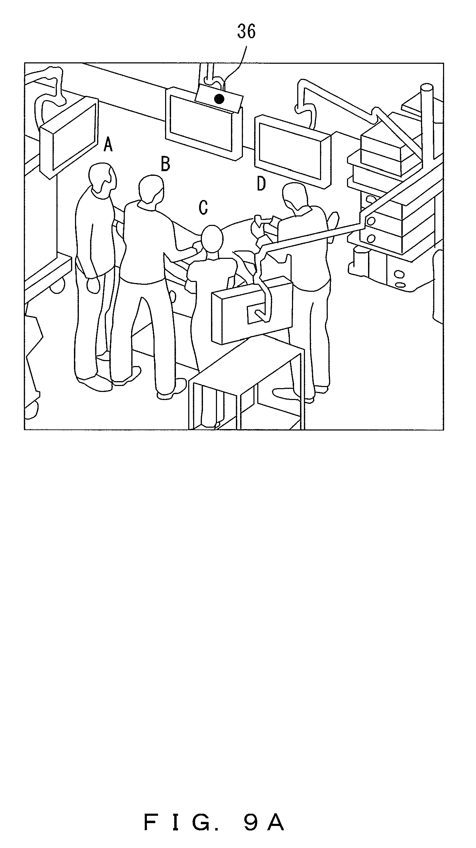

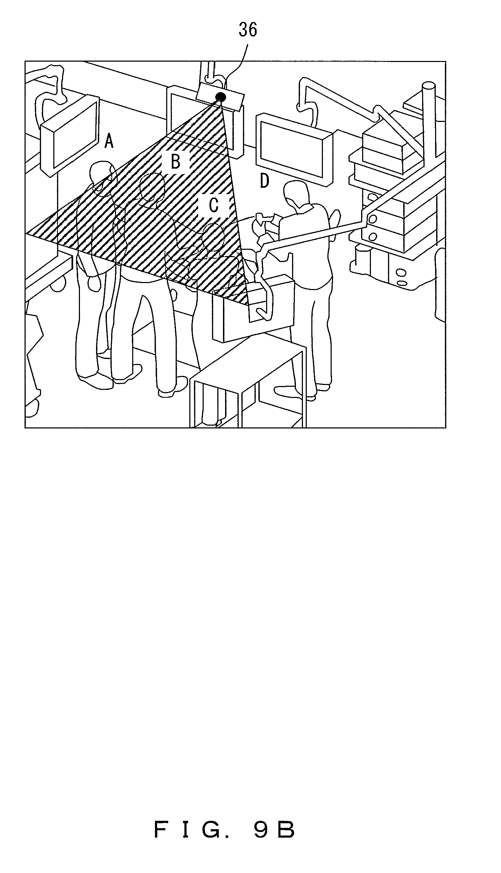

Next, by referring to FIGs. 8, 9A and 9B, specific explanations will be given for the flow of a process of controlling a device by recognizing gestures. FIG. 8 is a flowchart explaining a device control process for the medical endoscope system according to the present example. FIG. 9A shows a scene in the surgery room 2 when the device control process exemplified in FIG. 8 is being performed. FIG. 9B shows an image pickup scope of the gesture camera included in the medical endoscope system according to the present example.

In step S10, the gesture camera 36 picks up an image of a scene inside the surgery room 2 where there are a plurality of surgeons (surgeons A, B, C, and D) as exemplified in FIG. 9A, and outputs a generated image to the system controller 22.

Conditions for detecting gesture actions of a surgeon are that the upper body including at least the face of the surgeon is in the image pickup scope of the gesture camera 36 and that even when the surgeon stretches his or her arms from side to side, the hands are still in the image pickup scope. Accordingly, it is desirable that the gesture camera 36 be disposed in such a manner that these conditions are met. In a case, for example, where the image pickup scope of the gesture camera 36 is the shaded portion in FIG. 9B, when the manipulator is surgeon B or C, gesture actions can be detected normally. Also, when gesture actions include actions of a lower body such as legs or the like, the gesture camera 36 is disposed in such a manner that the image pickup scope of the gesture camera 36 includes the lower bodies of surgeons. In this example, it is assumed that the gesture camera 36 is disposed in such a manner that the surgeon room is included entirely in the image pickup scope.

In step S11, the object detection unit 51 refers to the model information 65 stored in the storage unit 62, and detects a surgeon by detecting the face of a person meeting prescribed conditions set in the object setting unit 56, from an image output from the gesture camera 36. In this example, it is assumed for example that the face of surgeon B in the surgery room 2 is detected so that surgeon B is detected.

In step S12, the manipulator recognition unit 52 compares the image of the face of surgeon B detected from an image output from the gesture camera 36 with the face data of surgeon A that is stored in the storage unit 62 and that is set as a manipulator by the manipulator setting unit 57. In the comparison process, various types of information (relative positions of parts of faces, the sizes of eyes, the sizes of noses, etc.) that are stored in the storage unit 62 may be used together with the face images. On the basis of the result of this, the manipulator recognition unit 52 determines whether or not the detected surgeon is identical to the manipulator (step S13). In this example, it has been determined in step S13 that the detected surgeon is not identical to the manipulator, and accordingly the process returns to step S11 so that the processes from step S11 through step S13 are repeated.

When the image of the face of surgeon A is detected in the second object detection process (step S11), the manipulator recognition unit 52 determines in step S13 that the detected surgeon is identical to the manipulator, and recognizes the detected surgeon as the manipulator in step S14. Thereby, the manipulator recognition unit 52 determines surgeon A so as to recognize surgeon A as the manipulator.

In steps S15 through S18, the action detection unit 53 monitors actions of surgeon A determined by the manipulator recognition unit 52, through an image output from the gesture camera 36, and thereby detects a prescribed gesture action of surgeon A set in the action setting unit 59.

Specifically, the action detection unit 53 refers to, in step S15, the model information 65 of respective portions stored in the storage unit 62, and detects, as shown in FIG. 4, respective portions of the body of surgeon A, who is the manipulator determined by the manipulator recognition unit 52, from an image output from the gesture camera 36. In step S16, the action detection unit 53 estimates the posture of the manipulator from the detected respective portions so as to form a frame model of a human body. In step S17, the action detection unit 53 tracks the motion of the frame model formed from images output from the gesture camera 36 on an as-needed basis, and detects actions of the manipulator. In step S18, the action detection unit 53 refers to the gesture information 66 stored in the storage unit 62 to so as to determine whether or not the detected action is a gesture action. When the detected action has been determined to be a gesture action, the process in step S19 is executed, while when the detected action has been determined to not be a gesture action, the process returns to step S11, and the above process is repeated.

In step S19, the control signal generation unit 54 generates a control signal for controlling a control target device in accordance with the gesture action detected by the action detection unit 53, and outputs the signal to the device control unit 55.

In step S20, the device control unit 55 controls the control target device in accordance with a control signal from the control signal generation unit 54. For example, when the action detection unit 53 has detected a gesture action of a hand moving from right to left, the light source device 16 is controlled in such a manner that the amount of light emitted from the light source device 16 decreases, and when a gesture action of a hand moving from left to right has been detected, the light source device 16 is controlled in such a manner that the amount of light emitted from the light source device 16 increases. Also, when the action detection unit 53 has detected a gesture action of a hand making a fist, the device control unit 55 controls the light source device 16 into an OFF state, and when the action detection unit 53 has detected a gesture action of a hand opening a fist, the light source device 16 is controlled into an ON state.

In the medical endoscope system 3 configured as described above, only surgeon A can control devices with gesture actions, and other surgeons B, C, or D cannot control devices with gestures. Accordingly, by appropriately setting the manipulator beforehand, it is possible to prevent a situation where the system controller 22 controls devices by reacting to motion of a surgeon that is not intended to manipulate a device even when there are a plurality of surgeons in the surgery room 2 as exemplified in FIG. 9A.

Also, in the setting of a manipulator, when surgeon A has been set as a manipulator, surgeons B, C, and D are automatically set as manipulation prohibited persons. This can prevent a situation where a plurality of surgeons are set as manipulators by mistake.

Accordingly, the medical endoscope system 3 makes it possible to permit only a particular surgeon to manipulate a device with gestures while preventing other surgeons from manipulating devices with gestures, and as a result of this, it is possible to eliminate, at a sufficiently high level required for medical endoscope systems, a situation in which devices are controlled on the basis of mistaken recognition.

### Example 2

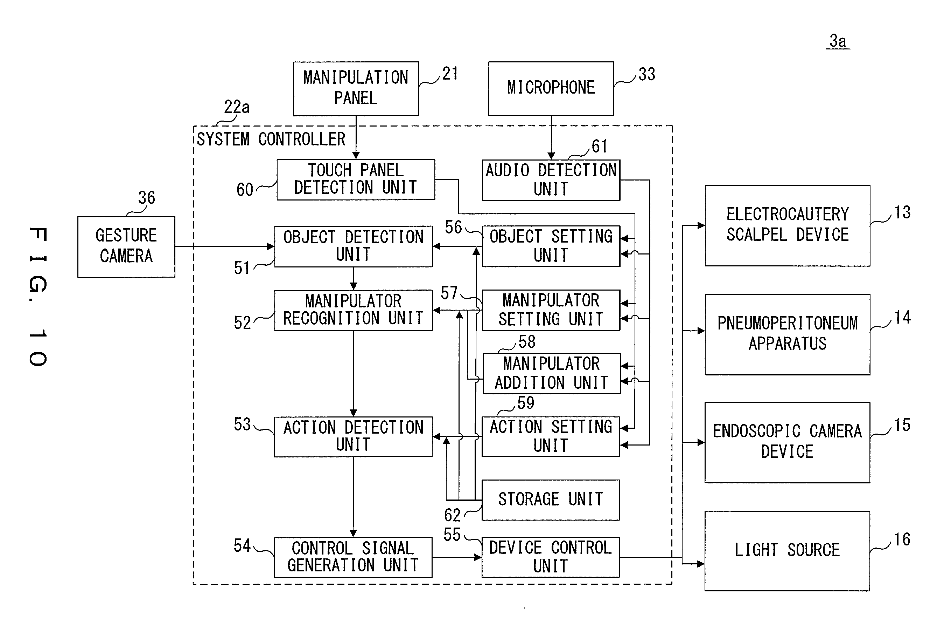

FIG. 10 shows a configuration of a system controller included in the medical endoscope system according to the present example.

A system controller 22a exemplified in FIG. 10 includes a manipulator addition unit 58, which is different from the system controller 22 according to example 1 exemplified in FIG. 2. The system controller 22a is similar to the system controller 22 in other points, and accordingly the same constituent elements are denoted by the same symbols, omitting the explanations thereof. Also, a medical endoscope system 3a according to the present example includes the system controller 22a instead of the system controller 22, which is different from the medical endoscope system 3 according to example 1 exemplified in FIG. 1. The medical endoscope system 3a is similar to the medical endoscope system 3 in the other points.

The manipulator addition unit 58 is configured to change a manipulation prohibited person set by the manipulator setting unit 57 into a manipulator. Accordingly, in the medical endoscope system 3a, the manipulator recognition unit 52 determines a surgeon who corresponds to the manipulator set by the manipulator setting unit 57 and the manipulator addition unit 58 from among surgeons detected by the object detection unit 51, and recognizes the determined surgeon as a manipulator.

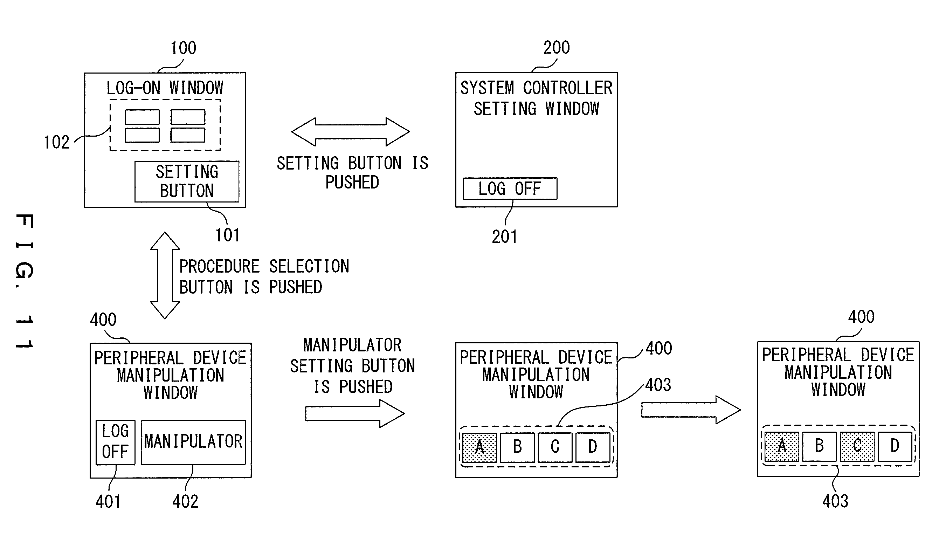

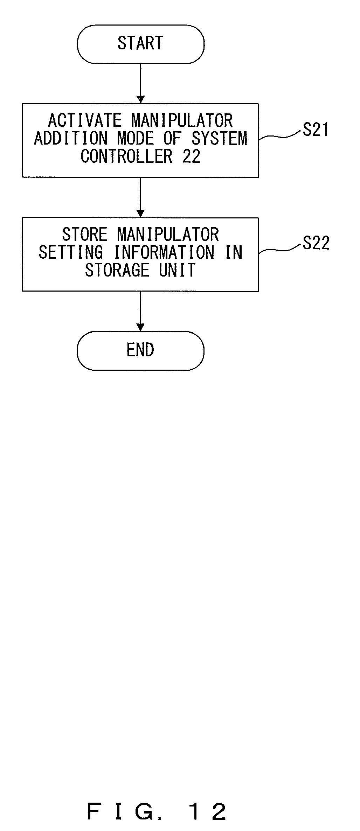

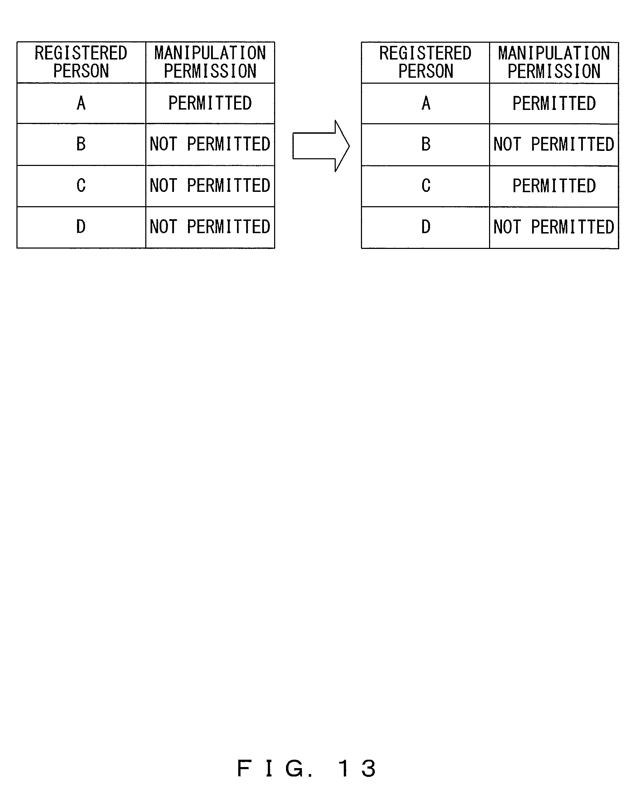

By referring to FIGs. 11 through 13, specific explanations will be given for the flow of a process of adding a manipulator. FIG. 11 shows window transition in a manipulator addition process for the medical endoscope system according to the present example. FIG. 12 is a flowchart showing the manipulator addition process for the medical endoscope system according to the present example. FIG. 13 shows an example of a setting state of manipulators before and after the manipulator setting process exemplified in FIG. 12.

The manipulator addition process starts when a manipulator setting button 402 disposed on a peripheral device manipulation window 400 is pushed while the peripheral device manipulation window 400 exemplified in FIG. 11 is displayed on the manipulation panel 21. The peripheral device manipulation window 400 is provided with a log-off button 401 in addition to the manipulator setting button 402. When the log-off button 401 is pushed, the window transitions to the log-on window 100.

When the manipulator setting button 402 is pushed on the peripheral device manipulation window 400, the manipulator addition mode of the system controller 22 is activated (step S21 in FIG. 12).

When the manipulator addition mode is activated, a manipulator button group 403 in which only the button of surgeon A, currently set as a manipulator, is in an ON state (shaded in the figure) is displayed at a lower portion of the peripheral device manipulation window 400.

The manipulator button group 403 is set to disappear when a prescribed period of time (3 seconds, for example) has elapsed. By selecting the button of a surgeon of whom the addition as a manipulator is desired while the manipulator button group 403 is displayed, the manipulator addition unit 58 changes the setting of manipulators in accordance with the input, and the manipulator setting information after the change is stored in the storage unit 62 (step S22 in FIG. 12). Thereby, when, for example, the button of surgeon C is selected, as shown in FIG. 13, the setting state of manipulators registered in the storage unit 62 changes from a state in which only surgeon A is set as a manipulator to a state in which two surgeons, i.e., surgeons A and B are set as manipulators.

According to the medical endoscope system 3 of example 1, it is necessary to cause window transition from the peripheral device manipulation window 400 to the system controller setting window 200 through the log-on window 100 in order to change the setting of manipulators, and by contrast, according to the medical endoscope system 3a, it is possible to add a manipulator through the peripheral device manipulation window 400 that is being displayed during a surgery. This makes it possible to add a surgeon easily and promptly with fewer input operations in an exceptional case where a manipulator has to be added during a surgery. As examples of exceptional cases where a manipulator has to be added, there is a case where the manipulator has objects in both hands and thus he or she cannot make gesture actions, raising the need for other surgeons to control devices. There is also a case where a surgeon who is giving training to a less experienced surgeon has to control a device, and the like.

A situation where a manipulator has been added so that a plurality of manipulators are set is only admitted as an exceptional case, and thus the setting performed by the manipulator addition unit 58 is distinguished from the setting performed by the manipulator setting unit 57. Specifically, the setting by the manipulator setting unit 57 is maintained unless the setting is changed again by the manipulator setting unit 57. In other words, the setting is maintained even when a peripheral device manipulation window for a different peripheral device is displayed or the system controller 22 is turned off. By contrast, the setting by the manipulator addition unit 58 is deleted when the log-off button 401 is pushed on the peripheral device manipulation window 400 so that the window has transitioned to the log-on window 100.

Therefore, according to the medical endoscope system 3a, in a basically similar manner to the medical endoscope system 3 of example 1, it is possible to permit only one particular surgeon to manipulate a device with gestures and prohibit other surgeons from manipulating a device with gestures, and as a result of this, it is possible to eliminate, at a sufficiently high level required for medical endoscope systems, a situation in which devices are controlled on the basis of misrecognition.

Further, according to the medical endoscope system 3a, it is possible to add a manipulator with simple input so that a plurality of surgeons are set as manipulators in an exceptional case, allowing a surgeon other than a manipulator to control devices with gestures, which makes surgeries progress without delay even when the primary manipulator cannot perform gesture manipulation. Also, the addition is treated as an exception, preventing a permanent setting situation where there are a plurality of manipulators.

An example of adding a manipulator through the peripheral device manipulation window 400 has been described above, however, it is also possible to perform a process of deleting a manipulator through the peripheral device manipulation window 400. It is also possible to change a manipulator to a manipulation prohibited surgeon when a button that has already been set as a manipulator in the manipulator button group 403 displayed on the peripheral device manipulation window 400 is pushed. Thereby, manipulators can be changed arbitrarily on the peripheral device manipulation window 400.

Also, an example of adding a manipulator through the peripheral device manipulation window 400 displayed on the manipulation panel 21 has been explained above, however, the adding process of a manipulator may be performed by a gesture by using an image output from the gesture camera 36 similarly to the control of devices. Also, the process may be performed by inputting audio information by using the microphone 33.

It is possible to employ a configuration in which, depending upon setting, the manipulator setting button 402 displayed on the peripheral device manipulation window 400 is not displayed. Thereby, only changing of the setting causes the same effect as caused by a configuration of switching between the medical endoscope system 3 and the medical endoscope system 3a.

### Explanations of Symbols

- 2: surgery room
- 3, 3a: medical endoscope system
- 4: patient monitor system
- 9: cable
- 10: patient bed
- 11, 12: cart
- 13: electrocautery scalpel device
- 14: pneumoperitoneum apparatus
- 15, 23: endoscopic camera device
- 16, 24: light source device
- 17: video tape recorder
- 18: gas tank
- 19, 26: display device
- 20, 27: concentrated display panel
- 21: manipulation panel
- 22, 22a: system controller
- 25: image processing device
- 28: relay unit
- 29: relay cable
- 30: remote controller
- 31, 32: endoscope
- 31a, 32a: camera cable
- 31b, 32b: light guide cable
- 33: microphone
- 35: RFID terminal
- 36: gesture camera
- 48: patient
- 51: object detection unit
- 52: manipulator recognition unit
- 53: action detection unit
- 54: control signal generation unit
- 55: device control unit
- 56: object setting unit
- 57: manipulator setting unit
- 58: manipulator addition unit
- 59: action setting unit
- 60: touch panel detection unit
- 61: audio detection unit
- 62: storage unit
- 63: manipulator setting information
- 64: face data
- 65: model information
- 66: gesture information
- 100: log-on window
- 101: setting button
- 102: procedure selection button group
- 200: system controller setting window
- 201, 301, 401: log-off button
- 300, 400: peripheral device manipulation window
- 402: manipulator setting button

## Claims

1. A medical endoscope system comprising:
an image pickup device, a plurality of control target devices, and a controller that controls the plurality of control target devices, wherein:
the controller comprises:
a manipulator setting unit configured to set one manipulator candidate as a manipulator who is permitted to perform manipulation from among a plurality of manipulator candidates registered beforehand, and to set other manipulator candidates other than the one manipulator candidate as a manipulation prohibited persons who are prohibited from performing manipulation;
an object detection unit configured to detect an object that meets a prescribed condition from an image output from the image pickup device;
a manipulator recognition unit configured to determine an object that corresponds to the manipulator set by the manipulator setting unit from among objects detected by the object detection unit, and to recognize the determined object as the manipulator;
an action detection unit configured to detect a prescribed action of the determined object;
a control signal generation unit configured to generate a control signal that controls at least one control target device among the plurality of control target devices in accordance with a result of detection by the action detection unit; and
a device control unit configured to control the at least one control target device in accordance with the control signal.

2. The medical endoscope system according to claim 1, wherein:
the controller includes a manipulator addition unit configured to change, to a manipulator, a manipulation prohibited person set by the manipulator setting unit.

3. The medical endoscope system according to claim 2, further comprising:
a display unit configured to display information about the medical endoscope system; and
an input unit configured to receive an input from outside of the medical endoscope system, wherein:
the manipulator addition unit is configured to change, to a manipulator, a manipulation prohibited person set by the manipulator setting unit in accordance with an input from the input unit in a situation where a window for manipulating each of the plurality of control target devices is displayed on the display unit.

4. The medical endoscope system according to claim 1, further comprising:
a storage unit configured to store model information of a portion of the object as the prescribed condition, wherein:
the object detection unit detects the object that meets the prescribed condition from an image output from the image pickup device by referring to the model information stored in the storage unit.

5. The medical endoscope system according to claim 4, wherein:
the storage unit further stores identification information for identifying the object; and
the manipulator recognition unit determines, by referring to the identification information stored in the storage unit and from among objects detected by the object detection unit, an object that corresponds to a manipulator set by the manipulator setting unit, and recognizes the determined object as the manipulator.

6. The medical endoscope system according to claim 5, wherein:
the storage unit further stores gesture information that is information related to the prescribed action; and
the action detection unit detects the prescribed action of the determined object by referring to the gesture information stored in the storage unit.
